# EUROPEAN PATENT APPLICATION

(11) **EP 3 842 552 A1**
(43) Date of publication of application: **30.06.2021**
(21) Application number: 20215357.3
(22) Date of filing: 18.12.2020
(51) Int. Cl.: C12Q 1/6883

(54) **KIT FOR IN VITRO TESTING PANEL OF GENES IN PAP SMEAR SAMPLES FOR ENDOMETRIOSIS AND METHOD OF NON-INVASIVELY AND QUALITATIVELY DETERMINING SEVERITY OF ENDOMETRIOSIS**

(30) Priority: 24.12.2019 US 201916726546
(71) Applicant: National Sun Yat-Sen University, Kaohsiung 804 (TW)
(72) Inventor: SHEU, Jim Jinn-Chyuan, Kaohsiung 804 (TW); CHANG, Yin-Yi, Kaohsiung 804 (TW); CHEN, Chih-Mei, Kaohsiung 804 (TW)
(74) Representative: dompatent von Kreisler Selting Werner - Partnerschaft von Patent- und Rechtsanwälten mbB

(57) **Abstract**

The present invention relates to a kit for in vitro testing a panel of genes in Pap smear samples for endometriosis and a method of non-invasively and qualitatively determining severity of endometriosis using the kit. A biomarker and a probe used in the kit and the method include a specific SNP site and a product expressed by a gene related to the SNP site, for detecting the genotype of SNP site and the product expressed by the gene corresponding to the SNP site, so as to accurately determine the severity of endometriosis. The biomarker and the probe can be applied to a kit for in vitro testing a panel of genes in Pap smear samples for endometriosis.

## Description

### BACKGROUND

### Field of Invention

The present invention relates to a biomarker of detecting a biological sample, a probe and its application. More specifically, the present invention relates to a biomarker of detecting a biological sample, a probe, a kit and a method of non-invasively and qualitatively determining severity of endometriosis.

### Description of Related Art

Endometriosis is a benign, yet debilitating, gynecological disease associated with chronic pelvic pain, dysmenorrhea and infertility. Affecting about l0% of reproductive-age females, endometriosis causes abnormal growth of endometrium-like tissues outside the uterine cavity. These benign peritoneal surface growths, which can invade ectopically, mimic the progression of metastasis in malignant cancer, which is accompanied by angiogenesis and cell migration. Histopathological observations and genetic analyses have shown that both endometrioid and clear cell ovarian carcinomas arise from endometriosis. Although several hypotheses have been proposed regarding the etiology of endometriosis, the exact pathogenesis of the disease remains unclear. Multiple factors may be involved in an individual's susceptibility to endometriosis, including hormone aberrations, abnormal immune responses, environmental factors and individual anatomy, as well as genetic or epigenetic predisposition.

MicroRNAs (miRNAs) are small non-coding single-stranded RNAs that post-transcriptionally regulate a wide range of biological processes, including cellular differentiation, proliferation and apoptosis. Targeting mRNA transcripts by miRNAs accelerates its transcript degradation or represses the translation, depending on the degree of complementarity. Single-nucleotide polymorphisms (SNPs) in miRNAs or their binding targets have been associated with aberrant miRNA expression and carcinogenesis. Microarray and functional studies revealed that miRNA levels are related to benign conditions, malignant diseases and fertility disorders of the female reproductive tract alike, but no link has yet been established between miRNA gene polymorphisms and endometriosis.

Small nucleolar RNAs (snoRNAs) are non-coding RNAs with longer mature sequences (60-300 nt) than miRNAs. They can be divided into two major classes with distinct signature sequences, box *C*/*D* or box *H*/*ACA,* functioning as guiding components for small ribonucleoprotein particles, catalyzing rRNA 2'-O-methylation and pseudouridylation, respectively, through complementary recognition sequences. In the eukaryotic cell nucleolus, ribosomal RNA is post-transcriptionally edited by snoRNAs and subsequently cleaved to yield 18S, 5.8S and 28S rRNAs. These fragments are assembled into the mature large and small RPs, preceding translocation to the cytoplasm. Both snoRNAs and RPs are key regulators in ribosome biogenesis, which is especially crucial for cell cycle progression. Recent studies suggested that upregulation of snoRNAs and RPs controls human tumor development. Perturbation of ribosome assembly by RNA polymerase I inhibition or snoRNA/RP silencing can arrest cell proliferation and induce apoptosis, and has been suggested as a novel strategy against malignant diseases.

However, there is no effective strategy to non-invasively determining severity of endometriosis. Accordingly, there is an urgent need to develop a novel strategy to beneficially determining severity of endometriosis.

### SUMMARY

The invention provides a biomarker of detecting a biological sample, which comprises a single-nucleotide polymorphism (SNP) site and at least one sequence epigenetically associated with the SNP site, thereby determining the severity of endometriosis in the biological sample.

Moreover, the invention also provides a method of qualitatively detecting an expression of a biomarker in a biological sample, which establishes an association model between the biomarker and severity of endometriosis, followed by determining an expression of the biomarker in a test biological sample according to the association model, thereby determining that the test biological sample has the one of the severe statuses.

Furthermore, the invention provides a probe of detecting an expression of a biomarker in a biological sample.

Still moreover, the invention provides a kit for in vitro testing a panel of genes in Pap smear samples for endometriosis, which comprises at least nine antibodies recognizing the nine genes as aforementioned.

According to the aforementioned aspect, the invention provides a biomarker of detecting a biological sample, which comprises a single-nucleotide polymorphism (SNP) site and at least one sequence epigenetically associated with the SNP site. In an embodiment, the SNP site has a SNP accession number of rs11614913 and/or rs1834306, and the SNP site has a genotype. In the embodiment, the at least one sequence can be SNORD genes and RP genes involved in ribosome biosynthesis, which can include but be not limited to a DNA sequence, a RNA sequence encoded by the DNA sequence and/or an amino acid sequence encoded by the RNA sequence, and the at least one sequence can be SNORD genes and RP genes involved in ribosome biosynthesis, which can be originated from SNORD116 gene, RPLP2 gene, RPL26 gene, RPL38 gene, RPS25 gene, RPS27 gene, and/or RPS28 gene, so as to determine that the test biological sample has the one of the severe statuses.

In the aforementioned embodiment, the genotype of the rs11614913 can include C allele, CC genotype or CT genotype.

In the aforementioned embodiment, the genotype of the rs1834306 can include A allele or AA genotype.

According to the another aspect, the invention provides a method of non-invasively and qualitatively determining severity of endometriosis, which includes steps of providing a kit for in vitro testing a panel of genes in the biological sample for endometriosis, establishing a correlation model, and determining an expression of the biomarker in a test biological sample according to the correlation model. In an embodiment, the kit includes at least nine antibodies, in which each of the nine antibodies respectively recognizes one protein expressed by the panel of genes or SNP sites, the panel includes at least nine different genes, and the panel is selected from the group consisting of SNORD116 gene, RPLP2 gene, RPL26 gene, RPL38 gene, RPS25 gene, RPS27 gene, RPS28 gene, rs11614913 and/ or rs1834306. In an embodiment, the step of establishing the correlation model includes detection of a plurality of reference biological samples by the kit, so as to obtain a plurality of first risk data; and establishment of a correlation between the first risk data and a plurality of severe statuses of endometriosis, so as to correspond one of the SNP sites with one of the genes or the proteins, one of the first expressions and/or one of the severe statuses. In the aforementioned embodiment, the first risk data can include a plurality of SNP sites and first expressions of a plurality of genes or proteins epigenetically associated with the SNP sites, in which SNP accession numbers of the SNP sites comprises rs11614913 and rs1834306, and the genes or the proteins are selected from the group consisting of SNORD116, RPLP2, RPL26, RPL38, RPS25, RPS27, and RPS28. When the second risk data matches the one of the SNP sites and the one of the genes or the proteins, and the corresponding significance difference (P value) is smaller than 0.05, the test biological sample can be determined to have the one of the severe statuses.

In the aforementioned embodiment, the reference biological samples and the test biological sample are Pap smear samples comprising an *ex vivo* sample of blood or tissue.

In the aforementioned embodiment, the first expressions are upregulated.

In the aforementioned embodiment, the severe statuses include a clinical stage, a CA125 level and a pain score of the reference biological sample.

According to the further aspect, the invention provides a probe of detecting an expression of a biomarker in a biological sample, which is characterized by the probe of detecting the aforementioned biomarker.

According to the still further aspect, the invention provides a kit for in vitro testing a panel of genes in Pap smear samples for endometriosis, which includes at least nine antibodies, each of the nine antibodies respectively recognizes one protein expressed by the panel of genes or SNP sites, the panel includes at least nine different genes, and the panel is selected from the group consisting of SNORD116 gene, RPLP2 gene, RPL26 gene, RPL38 gene, RPS25 gene, RPS27 gene, RPS28 gene, rs11614913 and/ or rs1834306.

With application to the biomarker of determining severity of endometriosis, which includes a specific SNP site and at least one sequence epigenetically associated with the SNP site. By establishing a correlation model between the biomarker and severe statuses of endometriosis, the degree of severity of endometriosis in the test biological sample can be accurately determined, thereby being applied to a probe and a kit for in vitro testing a panel of genes in Pap smear samples for endometriosis.

It is to be understood that both the foregoing general description and the following detailed description are by examples, and are intended to provide further explanation of the invention as claimed.

### BRIEF DESCRIPTION OF THE DRAWINGS

The disclosure can be more fully understood by reading the following detailed description of the embodiment, with reference made to the accompanying drawings as follows.
FIGS. 1A to 1D illustrate the risk analysis of cancer-related MiRSNPs for endometriosis and related clinical symptoms.
FIGS. 2A and 2B illustrate that *MIR196A2* genetic variant affects on RNA structures and downstream target gene expression.
FIGS. 3A to 3D illustrate that genetic variations at rs11614913 in *MIR196A2* lead to rRNA processing and protein synthesis malfunction in endometrial cells.
FIGS. 4A to 4D illustrate ribosome biogenesis upregulation during endometriosis progression.
FIGS. 5A to 10E show the expressions of nucleolin (FIGS. 5A to 5E), RPL38 (FIGS. 6A to 6E), RPS13 (FIGS. 7A to 7E), RPLP2 (FIGS. 8A to 8E), RPS27 (FIGS. 9A to 9E) and RPS25 (FIGS. 10A to 10E) in the cervix and vagina of patients and healthy women according to an embodiment of the present invention.
FIGS. 11A to 13 illustrate the cells expressing biomarker-positive cells per slide (FIGS. 11A to 11F), VAS pain scores related with the biomarkers (RPS27 and RPL38) (FIG. 12A), different disease stages related with the cells expressing biomarker (RPS27, RPLP2 and RPL38)- positive cells (FIG. 12B) and the CDS obliteration with the biomarkers (RPS25 and RPL38) (FIG. 13) between the endometriosis patients and healthy (normal) women.

### DETAILED DESCRIPTION

Reference will now be made in detail to the present embodiments of the invention, examples of which are illustrated in the accompanying drawings. Wherever possible, the same reference numbers are used in the drawings and the description to refer to the same or like parts.

As aforementioned, the present invention provides a biomarker, a probe, primer pairs, a kit and a method of non-invasively and qualitatively determining severity of endometriosis, for detecting the genotype of SNP site and the product expressed by the gene corresponding to the SNP site, so as to accurately determine the severity of endometriosis.

Typically, the "biomarker" as discussed hereinafter can include a single-nucleotide polymorphism (SNP) site and at least one sequence epigenetically associated with the SNP site. Epigenetics most often involves changes that affect gene activity and expression, but the term can also be used to describe any heritable phenotypic change. Such effects on cellular and physiological phenotypic traits may result from external or environmental factors, or be part of normal development. The standard definition of epigenetics requires these alterations to be heritable in the progeny of either cells or organisms. The term also refers to the changes themselves: functionally relevant changes to the genome that do not involve a change in the nucleotide sequence. These epigenetic changes may last through cell divisions for the duration of the cell's life, and may also last for multiple generations, even though they do not involve changes in the underlying DNA sequence of the organism; instead, non-genetic factors cause the organism's genes to behave (or "express themselves") differently. In an embodiment, the SNP site can has a SNP accession number of rs11614913 and/or rs1834306. In this embodiment, the SNP site has a genotype, in which the genotype of the rs11614913 includes C allele, CC genotype or CT genotype, and the genotype of the rs1834306 can include A allele or AA genotype. In other embodiment, the SNP site further optionally includes rs2910164, rs7372209, rs895819, rs6505162 and rs3746444 for combined analysis.

In the aforementioned embodiments, the at least one sequence can include but be not limited to DNA sequence, a RNA sequence encoded by the DNA sequence and/or an amino acid sequence encoded by the RNA sequence. In an example, the at least one sequence can be SNORD genes and RP genes involved in ribosome biosynthesis, being originated from small nucleolar RNA C/D box 116 (SNORD 116) gene, ribosomal P protein) 2 (RPLP2) gene, RPL26 gene, RPL38 gene, ribosomal protein) S25 (RPS25) gene, RPS27 gene, and/or RPS28 gene. It should be noted that, the aforementioned genes are merely illustrative and are not intended to limit the present invention.

The aforementioned biomarker can be further applied to non-invasively detection of qualitatively detecting an expression of a biomarker in a biological sample. The "biological samples" as discussed hereinafter denotes to include reference biological samples and test biological samples, such as an *ex vivo* sample of blood or tissue. The "reference biological sample" is used for establishment of the correlation model, thereby subsequently determining the severe statues of endometriosis in the test biological sample.

The reference biological samples and the test biological sample comprises an *ex vivo* sample of blood or tissue.

In detail, in an embodiment, the method of non-invasively and qualitatively detecting a panel of genes in a biological sample can include provision with a kit for in vitro testing a panel of genes in the biological sample for endometriosis, establishment of a correlation model, and determination of an expression of the biomarker in a test biological sample according to the correlation model.

In the aforementioned embodiment, the step of establishing the correlation model includes detection of a plurality of reference biological samples, so as to obtain a plurality of first risk data. In an embodiment, the first risk data can include a plurality of SNP sites and first expressions of a plurality of genes or proteins epigenetically associated with the SNP sites. In an example, SNP accession numbers of the SNP sites can include rs11614913 and rs1834306. In another example, suitable examples of the genes or the proteins can include but be not limited to SNORD116, RPLP2, RPL26, RPL38, RPS25, RPS27, and RPS28. In this example, the first expressions of the genes or the proteins are usually upregulated, for enhancing ribosome biosynthesis.

After obtaining the first risk data, the establishment of the correlation model further includes establishment of a correlation between the first risk data and a plurality of severe statuses of endometriosis, so as to correspond one of the SNP sites with one of the genes or the proteins, one of the first expressions and/or one of the severe statuses. For example, C allele at rs11614913 is correlated with infertility and increased pain, and expressions of SNORD 116, RPLP2, RPL38 and RPS28 are higher. In another example, A allele at rs1834306 is correlated with infertility and advanced endometriosis stage. Those examples are merely to illustrate the correlation between the first risk data and severe statuses of endometriosis, rather than being limited thereto.

Next, an expression of the biomarker in a test biological sample is determined according to the correlation model. In an embodiment, the step of determining an expression of the biomarker in a test biological sample includes non-invasive detection of the test biological sample, so as to obtaining a plurality of second risk data. And then, the second risk data is compared to the first risk data, for determining that the second risk data matches one of the first risk data or not, and obtaining a corresponding significance difference (P value).

The test biological sample is considered to have the one of the severe statuses when the second risk data matches the one of the SNP sites and the one of the genes or the proteins, and the corresponding significance difference (*P* value) is smaller than 0.05.

For example, when the second risk data matches the C allele at rs11614913, and the corresponding P value is smaller than 0.05, the test biological sample is considered to have the risk of infertility and increased pain.

The "risk allele" as discussed hereinafter is defined as alleles that elevate the risk of the disease influencing an individual, and the odds ratio (OR) or minor allele frequencies (MAF) of the allele to the specific disease are positively correlated.

The "protective allele" as discussed hereinafter is defined as alleles that confer protection against disease by disrupting protein function or attenuate disease risk.

The "upregulated" or "upregulation" as discussed hereinafter denotes to increase the amount of particular cellular components such as DNA, RNA protein and the like. On the contrary, the "downregulated" or "downregulation" as discussed hereinafter denotes to decrease the amount of particular cellular components such as DNA, RNA protein and the like.

It should be supplemented that, in other embodiments, a plurality of the first risk data can be combined and subjected to the risk analysis, so as to further evaluate the risk of severe statuses of endometriosis.

Thereinafter, various applications of the biomarker, the probe, the primer pairs, the kit and the method of non-invasively and qualitatively determining severity of endometriosis will be described in more details referring to several exemplary embodiments below, while not intended to be limiting.

### EXAMPLE 1

### 1. Establishment of Study population

In this example, the study population consisted of 218 individuals who were pathologically diagnosed with endometriosis and underwent laparotomy or laparoscopy at the China Medical University Hospital (CMUH) in Taiwan. Patient disease-related fertility statuses were verified by clinical reports. Endometriosis stages were classified using the guidelines of the American Society of Reproductive Medicine (ASRM): stage 1, minimal; stage 2, mild; stage 3, moderate; stage 4, severe. The control group consisted of 202 healthy women age-matched to the patient group, and received regular physiological examinations at the same hospital. People who showed ovarian cysts detected by ultrasound or anyone of the endometriosis-associated symptoms, even though the results of their health checkups were normal, were excluded from this study. This study was approved by the Institutional Review Board (IRB) at the CMUH, with informed consent from each participant.

### 2. Genotyping of single nucleotide polymorphisms

In this Example, genomic DNA was extracted from peripheral blood leukocytes or cell pellets according to standard protocols (Genomic DNA kit; Qiagen, Valencia, CA, USA). DNA fragments containing the SNP sites were amplified by PCR using the *Taqman* SNP genotyping assay system (Applied Biosystems Inc. Carlsbad, CA, USA) as previously described. Probe IDs for the six selected SNPs are listed in Table 1. A perfect match between the probe and the tested DNA fragment generated a positive signal. Genetic variations were detected via the fluorescence signals of PCR products. Each probe or primer comprises at least 15 nucleotides of complementary sequence to one of the panel of genes or SNP sites.

**Table 1: Summary of the six cancer-related MiRSNPs**

| SNP | Location in miRNA | Associated cancer type | Reference (PMID) | Allele frequencies^{a} (%) | ABI probe ID | Probe sequence | SEQ ID NO. |
|---|---|---|---|---|---|---|---|
| rs1834306 | Pri-miR-100 | Colon | 20585341 | A: 43 G: 57 | C_11483095_10_F | AGCAATATCTTCTATGTTCTCCCCA[A/G] CGTGCTTCCCTTGGTTTCCACTTTT | 2 |
| rs2910164 | Pri-miR-146a | Gastric | 22455393 | G: 43 C: 57 | C_15946974_10 | CATGGGTTGTGTCAGTGTCAGACCT[C/G] TGAAATTCAGTTCTTCAGCTGGGAT | 3 |
| | | Lung | 22818121 | | | | |
| | | Breast ^{a}/ovarian | 18660546 | | | | |
| rs11614913 | Pri-miR-196a2 | Breast ^{a} | 19567675 | C: 45 T: 55 | C_31185852_10 | TTTTGAACTCGGCAACAAGAAACTG[C/T] CTGAGTTACATCAGTCGGTTTCGT | 1 |
| | | Lung | 19293314 | | | | |
| | | Liver | 21692953 | | | | |
| rs7372209 | Pri-miR-26a1 | Colon | 20585341 | T: 32 C: 68 | C_29123986_10 | AGAATTAGGAGAGAAATTAATCCTT[C/T] GTACCACGTGACTGTAAGCATGACT | 4 |
| rs895819 | Pri-miR-27a | Gastric | 20666778 | C: 29 T: 71 | C_11483095_10_F | AGCAATATCTTCTATGTTCTCCCCA[A/G] CGTGCTTCCCTTGGTTTCCACTTTT | 5 |
| | | Breast | 19921425 | | | | |
| rs6505162 | Pri-miR-423 | Breast | 22593246 | A: 16 C: 84 | C_11613678_10 | TGAGGCCCCTCAGTCTTGCTTCCTA[A/C] CCCGCGCTTGAGTTTCTCCCCGCTT | 6 |

| | | | | | | | |
|---|---|---|---|---|---|---|---|
| ^{a}Allelic type frequencies provided by HapMap database (www.hapmap.org) for Han Chinese in Beijing(CHB). | | | | | | | |

### 3. Statistical analysis

Allelic and genotypic frequency distributions for the six SNPs in patients and controls were determined by chi-square analysis using SPSS software (version 10.0, SPSS Inc. Chicago, IL, USA) and expressed as percentages of the total number of alleles and genotypes. Odds ratios (ORs) were calculated for allelic and genotypic frequencies with 95% confidence intervals (95% CIs), using the most frequent allele as the reference. Combined risk analysis and differences between different drug/vector-treated cells were assessed via one-way ANOVA. Simple t-test was used to evaluate whether two groups with different treatments are equal or not.

### 4. Cell culture, gene transfection, cell sorting and functional study

Endometrial cells (HECIA and RL95-2) and ovarian clear cells (ES-2 and TOV-21G) were purchased from Bioresource Collection and Research center (BRCR), Taiwan. Vector pCMV-MIR (Origene, Rockville, MD) containing a green fluorescein protein (GFP) reporter gene was used to construct the miR196a2-C plasmid. A mutation (C to T) was introduced into the miR196a2-C plasmid at rs11614913 to generate the miR196a2-T using the QuikChange II site-directed mutagenesis kit (Agilent Technologies Inc., Santa Clara, CA). Sequences of resulting vectors were verified by direct sequencing (not shown).

For microarray analysis, 5x10⁵ HECIA endometrial cells were seeded in 6-cm dishes. Plasmids were introduced into cells using Lipofectamine (Invitrogen, Waltham, MA) as per the manufacturer's protocol. G418 at a final concentration of 200 ug/ml was added to culture medium 24 h post-transfection to select for positively transfected cells. At 48 h post-transfection, positive cells were sorted by GFP level via flow cytometry (Becton Dickinson, San Jose, CA), such that over 90% of cells were positively transfected. Sorting efficiencies were checked by counting fluorescent cells under a microscope.

For anti-ribosome biogenesis assays, including cell growth, cell migration and cell cycle analysis, ovarian clear cells were maintained for five days in culture medium with or without RNA polymerase I inhibitor, CX5461 (Selleckchem, Houston, TX).

### 5. Microarray experiment

Total RNA was prepared from sorted cells with TRIzol Reagent (Invitrogen) following the manufacturer's protocol. RNA quality was assessed using the Agilent Bioanalyzer (Agilent Technologies). Total RNA from each sample was processed for reverse transcription and fragmentation, followed by hybridization onto a GeneChip® human gene 1.0 ST Array (Affymetrix Inc, Santa Clara, CA). Gene chips were scanned after the wash step, and raw gene expression data in the generated CEL files were normalized and processed using the dChip algorithm. Further clustering and visualization were performed using the TM4 algorithm. Quantitative PCR analysis was performed to validate microarray data using the same RNA samples. To study clinical relevance, the microarray dataset (accession number: GSE6364) that comprises gene expression profiles of 16 normal endometriums and 21 endometriosis lesions was downloaded from the GEO databank (http://www.ncbi.nlm.nih.gov/gds). Data mining was performed by normalizing expression levels of a selected gene in normal endometriums as 1.0.

### 6. Immunofluorescence staining

Eight paraffin blocks showing continuous histopathological transition from distant endometriosis, contiguous atypical endometriosis and ovarian clear cell carcinomas were selected for sectioning. In this Example, five of the blocks were genotyped as carrying the *C*/*C* genotype, three as carrying T/T genotype at rs11614913 in *MIR196A2,* and were utilized for this study. Immunofluorescence staining was performed to detect active cell nucleoli and ribosome biogenesis activity using 1:100 rabbit anti-nucleophosmin (anti-NPM; ab52644) and anti-nucleolin (anti-NCL; ab129200) monoclonal antibodies (Abeam PLC, Cambridge, MA). Immunostaining was independently scored by two pathologists, and specific nucleolus staining was scored as: negative (0), weakly positive (1+), moderately positive (2+) or strongly positive (3+). This Example used a combination of the percentage of positively stained cells and the intensity of nucleolus staining for statistical analysis. The H-score = IPi xi was calculated, where i is the intensity of the stained tumor cells (0 to 3+) and Pi is the percentage of the stained tumor cells for each intensity group (0 to 100%) as disclosed in the Journal of Pathology 229:559-568 (2013), which is incorporated herein by reference. For discordant cases, a third investigator was brought in to score and the final intensity score was determined by the majority scores.

### Example 2: Risk association analysis of cancer-related SNPs in miRNA genes (MiRSNPs)

Six non-redundant SNPs within miRNA regions, also known as MiRSNPs, with minor allele frequencies over 4% in the Han Chinese in Beijing (HCB) population (HapMap database: www.hapmap.org) were selected. These MiRSNPs function as risk factors for various cancer types (Table 1). They are located within either pre- or mature miRNAs and could thus interfere with stability and folding. Data of this Example indicate that genetic variations at rs1834306 *in MIR100* (p=3.5 x10⁻³, OR: 1.64; 95% CI: 1.24-2.17) and rs11614913 in *MIR*/*96A2 (p=3.5x* 10⁻³, OR: 1.65; 95% CI: 1.24-2.19) are associated with endometriosis risk (Table 2). The rs11614913 C allele appears to dominantly affect endometriosis susceptibility; patients with CC or CT genotypes are at increased risk for endometriosis (*p*=7x 10⁻⁴, OR: 2.45; 95% CI: 1.54-3.51). The rs1834306 A allele recessively affects endometriosis susceptibility *(p*=9.1X 10⁻³, OR: 2.17; 95% CI: 1.35-3.51) (Table 3). Although the rs7372209 T allele in *MIR26AI* was also associated with increased endometriosis risk (Table 2), and the reference C allele was protective against endometriosis development (Table 3), these differences were not significant after Bonferroni correction.

**Table 2. Allele distributions of cancer-related MiRSNPs in Taiwanese patients with endometriosis and controls**

| miRNA | SNP | MAF^{a} | | OR^{b} | 95%CI^{c} | Nominal P-value^{d,f} | Corrected P-value^{e,f} |
|---|---|---|---|---|---|---|---|
| | | Patients (n=218) | Control (n=202) | | | | |
| miR-100 | rs1834306 | 46.76% | 34.83% | 1.64 | 1.24-2.17 | 0.0005*** | 0.0035** |
| miR-146a | rs2910164 | 39.79% | 34.83% | 0.80 | 0.47-1.38 | 0.49 | 1.00 |
| miR-196a2 | rs1161491 | 55.21% | 42.79% | 1.65 | 1.24-2.19 | 0.0005*** | 0.0035* |
| miR-26a1 | rs7372209 | 36.27% | 28.71% | 1.41 | 1.05-1.91 | 0.0232* | 0.1624 |
| miR-27a | rs895819 | 24.06% | 28.39% | 0.86 | 0.62-1.19 | 0.3594 | 1.00 |
| miR-423 | rs6505162 | 21.24% | 19.55% | 1.11 | 0.78-1.57 | 0.5541 | 1.00 |

| | | | | | | | |
|---|---|---|---|---|---|---|---|
| ^{a}MAF, minor allele frequency. ^{b}OR, odds ratio of minor alleles with reference to major alleles. ^{c}95% CI, 95% confidence interval. ^{d}*P* -values were calculated by chi-square tests. ^{e}Bonferroni method was applied for multiple test correction. ^{f}Statistical significance (*: *P* < 0.05; **: *P* < 0.01 ; ***: *P* < 0.001). | | | | | | | |

**Table 3. Genotype distributions of cancer-related MiRSNPs in Taiwanese patients with endometriosis and controls**

| SNP (miRNA) | Genotype | No.(%) of patients | No.(%) of control | P-value^{a,c} | Corrected P-value^{b,c} | OR (95%CI)^{d} |
|---|---|---|---|---|---|---|
| rs1834306 (miR-100) | AA | 63 (29.2) | 32 (15.9) | 0.0039** | 0.0273 | 2.38 (1.41-4.01) |
| | AG | 76 (35.2) | 76 (37.8) | | | 1.21 (0.78-1.87) |
| | GG | 77 (35.6) | 93 (46.3) | | | 1.00 |
| | AA+AG | 139 (64.4) | 108 (53.7) | 0.025* | 0.1918 | 1.55 (1.05-2.30) |
| | GG | 77 (35.6) | 93 (46.3) | | | 1.00 |
| | AA | 63 (29.2) | 32 (15.9) | 0.0013* | 0.0091** | 2.17 (1.35-3.51) |
| | GG+AG | 153 (70.8) | 169 (84.1) | | | 1.00 |
| rs2910164 (miR-146a) | GG | 38 (19.9) | 32 (15.9) | 0.4135 | 1.00 | 1.43 (0.82-2.51) |
| | AG | 76 (35.2) | 76 (37.8) | | | 1.21 (0.78-1.87) |
| | CC | 77 (40.3) | 93 (46.3) | | | 1.00 |
| | GG+CG | 114 (59.7) | 108 (53.7) | | 1.00 | 1.27 (0.85-1.90) |
| | CC | 77 (40.3) | 93 (46.3) | | | 1.00 |
| rs11614913 (miR-196a2) | CC | 55 (28.6) | 42 (20.9) | 0.0006*** | 0.0042** | 2.58 (1.47-4.58) |
| | CT | 102 (53.2) | 88 (43.8) | | | 2.35 (1.43-3.86) |
| | TT | 35 (18.2) | 71 (35.3) | | | 1.00 |
| | CC+CT | 157 (81.8) | 130 (64.7) | 0.0001*** | 0.0007*** | 2.45 (1.54-3.51) |
| | TT | 35 (18.2) | 71 (35.3) | | | 1.00 |
| rs7372209 (miR-26a1) | TT | 32 (16.6) | 17 (8.4) | 0.0419* | 0.2938 | 2.28 (1.19-4.39) |
| | CT | 76 (39.4) | 82 (40.6) | | | 1.12 (0.73-1.72) |
| | CC | 85 (44.0) | 103 (51.0) | | | 1.00 |
| | TT+CT | 108 (56.0) | 99 (49.0) | 0.0139** | 0.0971 | 1.32 (0.89-1.46) |
| | CC | 85 (44.0) | 103 (51.0) | | | 1.00 |
| | TT | 32 (16.6) | 17 (8.4) | 0.0139** | 0.0971 | 2.16 (1.16-4.04) |
| | CC+CT | 161 (83.4) | 185 (91.6) | | | 1.00 |
| rs895819 (miR-27a) | CC | 15 (8.0) | 16 (8.0) | 0.1962 | 1.00 | 0.85 (0.40-1.81) |
| | CT | 60 (32.1) | 81 (40.7) | | | 0.67 (0.44-1.04) |
| | TT | 112 (59.9) | 102(51.3) | | | 1.00 |
| | CC+CT | 75 (40.1) | 97 (48.7) | 0.0880 | 0.6158 | 0.70 (0.47-1.05) |
| | TT | 112 (59.9) | 102(51.3) | | | 1.00 |
| rs6505162 (miR-423) | AA | 12 (6.2) | 9 (4.5) | 0.7345 | 1.00 | 1.43 (0.58-3.51) |
| | AC | 58 (30.1) | 61 (30.2) | | | 1.02 (0.66-1.58) |
| | CC | 123 (63.7) | 132 (65.3) | | | 1.00 |
| | AA+AC | 70 (36.3) | 70 (34.7) | 0.4352 | 1.00 | 1.07 (0.71-1.62) |
| | CC | 123 (63.7) | 132 (65.3) | | | 1.00 |

| | | | | | | |
|---|---|---|---|---|---|---|
| ^{a}Genotype associations with endometriosis were determined by chi-square tests. ^{b}Bonferroni method was applied for multiple test correction. ^{c}Statistical significance (**: P* < 0.05 ; **: *P* < 0.01; ***: *P* < 0.001). ^{d}OR, odds ratio of minor alleles with reference to major alleles; 95% CI, 95%confidence interval. | | | | | | |

### 1. Association of MiRSNPs with clinical phenotypes

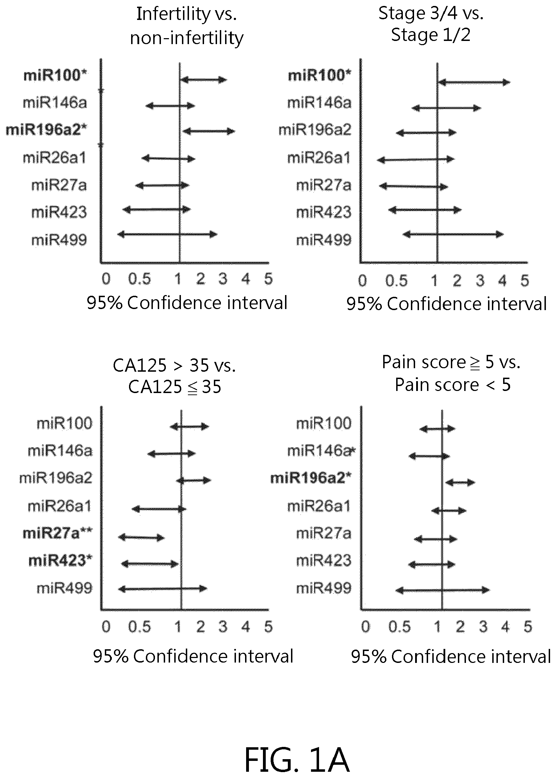

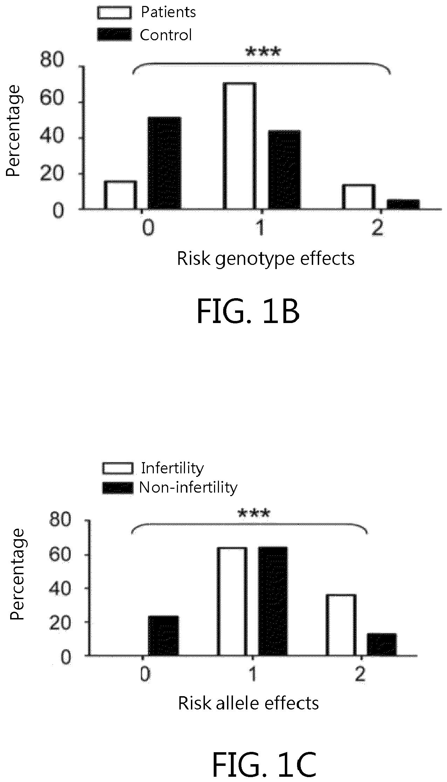

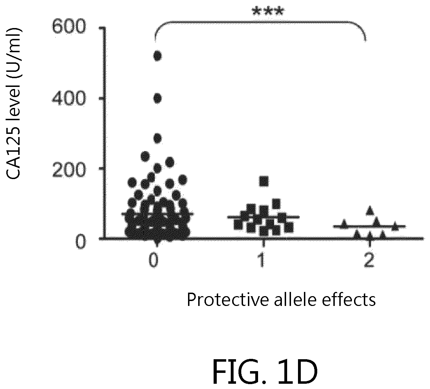

Please refer to FIGS. 1A to 1D, which illustrate the risk analysis of cancer-related MiRSNPs for endometriosis and related clinical symptoms. FIG. 1A is depicted to allele distributions of the defined MiRSNPs in patients were analyzed by chi-square tests and represented as 95% confidence intervals according to the indicated endometriosis-associated clinical symptoms. FIG. 1B is depicted to a combined genotype analysis of rs 11614913 (CC or CT genotype in *MIR196A2)* and rs1834306 (AA genotype in *MIR100)* was performed for endometriosis risk prediction. FIG. 1C is depicted to a combined allelic type analysis of rs 11614913 (C allelie in *MIR196A2)* and rs1834306 (A allele in *MIR100)* was performed to predict endometriosis-associated infertility. FIG. 1D is depicted to CA125 levels in patients with different protective allele effects were determined by combining rs8958l9 (C allele in *MIR2 7A)* and rs6505162 (A allele in *MIR423)* allelic types. FCombination effects in FIGS. 1B to 1D were labeled as 0: objectives with no risk or protective genotype/allelic type from either MiRSNP; 1: objectives with one risk or protective genotype/allelic type from either MiRSNP; 2: objectives with risk or protective genotype/allelic types from both MiRSNPs. **P<0.05; **P<0.01 ; ***P<0.001.*

Using patient records, a number of MiRSNPs linked to the development of endometriosis-associated phenotypes, including infertility, clinical stage, CA125 levels and pain scores (FIG. 1A) were discovered. The rs1834306 A allele in *MIR100,* which determines progression time in colon cancer, appeared linked to both infertility *(p=0.040)* and advanced endometriosis stage *(p=0.041)* (FIG. 1A). The rsl1614913 C allele in *MIR196A2* was involved in infertility *(p=0.016)* and increased pain severity *(p=0.012),* whereas SNP rs7372209 in *MIR26A1* was not associated with any clinical symptoms. The rs895819 in *MIR27A* and rs6505162 in *MIR423,* suggested to be protective alleles against endometriosis (Table 3), were linked with reduced CA125 levels *(p=0.0058* and 0.039, respectively; FIG. 1A). These data confirm the association of *MIR100* and *MIR196A2* genetic variants with endometriosis risk and cancer development.

The present application used a disease-associated genotype analysis to assess possible cumulative effects for the two pro-endometriosis functional SNPs, rs11614913 (CC or CT) of *MIR196A2* and rs1834306 (AA) of *MIR100.* Data indicated that patients and controls had distinct cumulative risk scores *(p<10⁻⁵;* FIG. 1B). Compared to low-risk patients with zero unfavorable genotypes, medium-risk patients with one unfavorable genotype had an OR of 5.31 (95% CI: 3.26-8.66), and high-risk patients with two unfavorable genotypes had an OR of 8.84 (95% CI: 4.06-19.2; Table 4). Similarly, a combination of the risk alleles at rsl16149l3 (C) in *MIR*/*96A2* and rs1834306 (G) in *MIR100* predicted endometriosis-related infertility (*p*<0.001; FIG. 1C). No patient with zero risk alleles developed infertility in our study group. By contrast, a combination of the minor allele frequencies in *MIR27A* and *MIR423* divided patients into three groups with different CA125 levels *(p*<*0*.001; FIG. 1D).

**Table 4. Combined risk analysis of endometriosis and the endometriosis-related infertility using MiRSNP markers**

| Association | Gene (risk genotype or allele) | Risk score | No.(%) of presence | No.(%) of absence | *P* -value | OR (95%CI) |
|---|---|---|---|---|---|---|
| Endometriosis | miR-196a2 (CC or CT) | 2 | 26 (13.5) | 10(5.0) | 4.1 x 10⁻¹³ | 8.84 (4.06-19.2 |
| | miR-100 (AA) | 1 | 136 (70.8) | 87 (43.7) | | 5.31 (3.26-8.66) |
| | | 0 | 30 (15.6) | 102(51.3) | | 1.00 |
| Infertility | miR-196a2 (C) | 2 | 18 (63.0) | 36 (12.6) | 1.4 x 10⁻³ | N/A |
| | miR-100 (A) | 1 | 32 (64.0) | 184 (64.3) | | N/A |
| | | 0 | 0(0.0) | 66 (23.1) | | 1.00 |

### 2. Variations at rs11614913 in MIR196A2 lead to rRNA editing/modification and protein synthesis malfunction in endometrial cells

Previous studies showed that MiRSNPs alter miRNA secondary structure and stability, resulting in gene expression and cellular signaling network changes, which may subsequently lead to cancer development. To address this, the present application used MaxExpect algorithm (http://ma.urmc.rochester.*edu*/*RN*Astructure*Web*/*Servers*/*MaxExpect*/*MaxExpe*c t.html) to predict possible structural changes in resulting pre-miRNAs and miRNAs. Although miRNA-100 is upregulated in endometriosis tissues compared with normal or eutopic endometrium, a recent study described its tumor-suppressive role in cancer through an untypical EMT process.

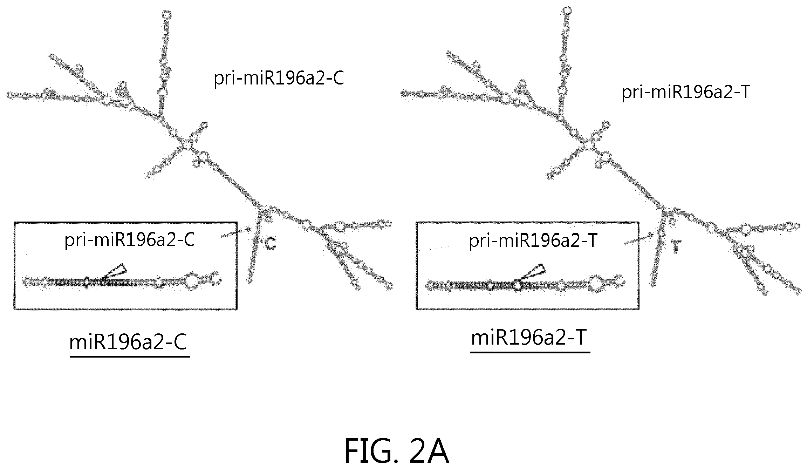

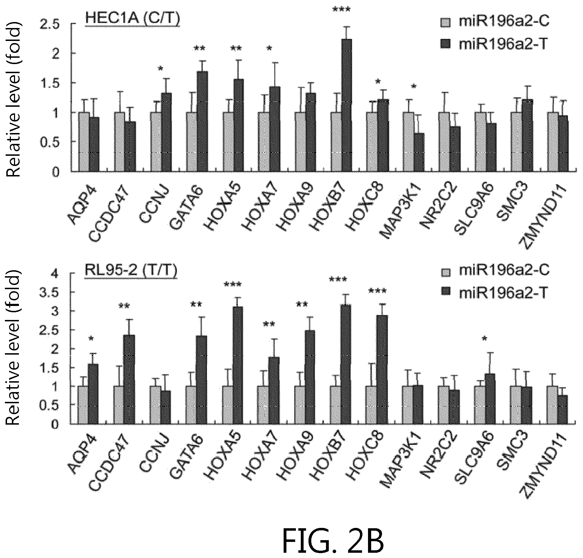

Please refer to FIGA. 2A and 2B, which illustrate that *MIR196A2* genetic variant affects on RNA structures and downstream target gene expression. The predicted pri-miRNA and pre-miRNA structures of miR196a2 with genetic variations at rs11614913 were analyzed by the MaxExpect algorithm. FIG. 2A is depicted to variant miR196a2-T showing an additional loop in the mature miR196a2 stem-loop structure (arrow head). FIG. 2B is depicted to quantitative PCR that was performed to compare mRNA levels of predicted miR196a2 downstream targets (Table 5) in endometrial HEC1A and RL95-2 cells transfected with miR196a2-C or miR196a2-T vectors. Data were represented as means of triplicates with standard variations. **P<0.05; **P<0.01* ; ****P<0.001.*

The present application therefore focused on the effects of genetic variations at rs11614913 in *MIR196A2* (FIG. 2A). Consistent with a previous study using free-energy analysis, a C to U(T) change in pre-miR196a2 generated an additional loop in the hairpin structure, leading to reduced stability and a smaller amount of the mature miR196a2. Quantitative PCR (qPCR) revealed that 6 of the 14 top-ranked target genes (Table 5) in HECIA (with a *T*/*C* genotype background) and 9 of the 14 genes in RL95-2 *(T*/*T* genotype) were upregulated in endometrial cells transfected with miR 196a2-T vector (T allele at rs11614913) as compared to cells transfected with miR196a2-C vector (C allele at rs11614913) (FIG. 2B), indicating insufficient silencing by the C to T substitution. Such target gene expression changes, although minimal, might alter downstream signaling.

**Table 5. The predicted downstream targets regulated by miR196a2^{a}**

| Target gene | RefSeq. | Gene description | miRDB^{b} | Target rank TargetScan^{c} | microRNA^{d} |
|---|---|---|---|---|---|
| AQP4 | NM_ 001650 | aquaporin 4 | 3 | 17 | NA |
| CCDC47 | NM_020198 | coiled-coil domain containing protein 47 | 12 | 15 | NA |
| CCNJ | NM_ 001134375 | cvclin J | 22 | 32 | 14 |
| GATA6 | NM_005257 | transcription factor GATA-6 | 6 | 20 | NA |
| HOXA5 | NM_ 019102 | homeobox protein Hox-A5 | 19 | 23 | NA |
| HOXA7 | NM_ 006896 | homeobox protein Hox-A7 | 17 | 2 | 1 |
| HOXA9 | NM_ 152739 | homeobox protein Hox-A9 | NA | 4 | 5 |
| HOXB7 | NM_ 004502 | homeobox protein Hox-B7 | 5 | 16 | NA |
| HOXC8 | NM_ 022658 | homeobox protein Hox-C8 | 10 | 1 | 2 |
| MAP3K1 | NM_005921 | mitogen-activated protein kinase kinase kinase 1 | 13 | 11 | 15 |
| NR2C2 | NM_003298 | nuclear receptor subfamily 2 group C member 2 | 4 | 12 | NA |
| SLC9A6 | NM_001042537 | sodium/hydrogen exchanger 6 | 2 | 3 | 4 |
| SMC3 | NM_005445 | structural maintenance of chromosomes protein 3 | 28 | NA | 3 |
| ZMYND 11 | NM_006624 | zinc finger MYND domain - containing protein | 1 | 8 | 9 |

| | | | | | |
|---|---|---|---|---|---|
| ^{a}The downstream targets of miR196a2 were predicted by overlapping the prediction results from three different algorithms. The genes that were ranked within the top-50 list by any two algorithms were considered as the potent target genes. ^{b}miRDB (http://mirdb.org/miRDB). ^{c}Targetxcan (http://www.targetscan.org). ^{d}microRNA (http://www.microrna.org/microrna/home.do). | | | | | |

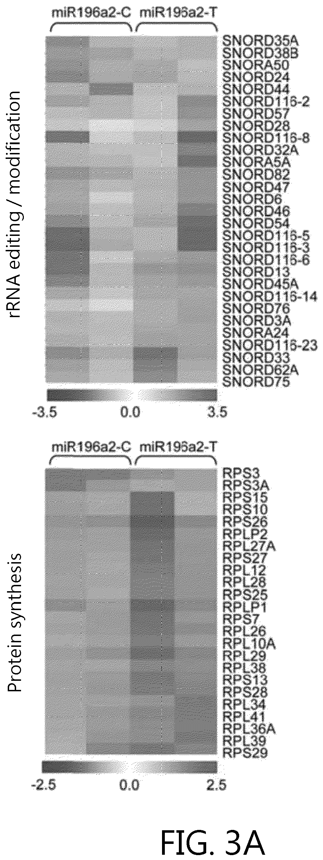

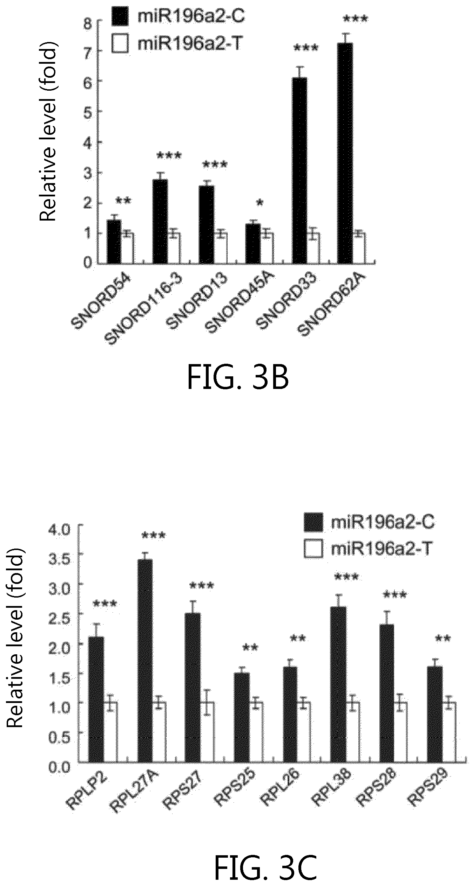

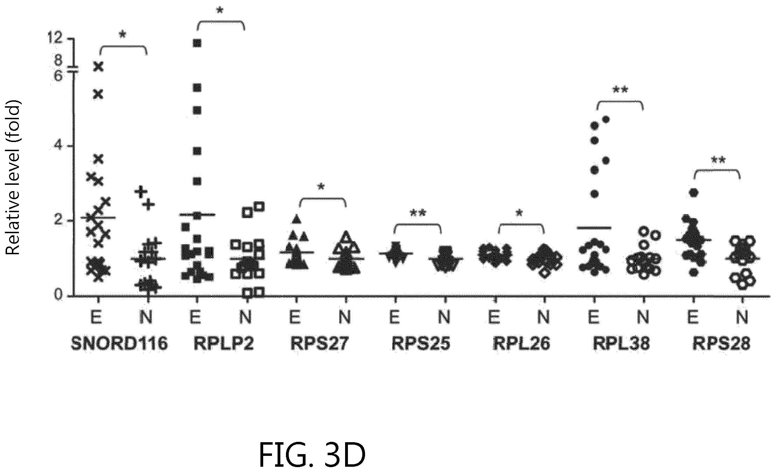

Please refer to FIGS. 3A to 3D, which illustrate that genetic variations at rs11614913 in *MIR196A2* lead to rRNA processing and protein synthesis malfunction in endometrial cells. Endometrial HEC 1A cells were transfected with miR196a2-C or miR196a2-T vectors. FIG. 3A is depicted to a microarray analysis showed that levels of snoRNAs (upper) and RPs (lower) were affected by rs11614913 variants. FIGS. 3B and 3C are respectively depicted to a quantitative PCR performed to validate expression of snoRNA (FIG. 3B) and RP (FIG. 3C) genes in transfected cells. Data were represented as means of triplicates with standard variations. FIG. 3D is depicted to microarray data from the GEO databank (GSE6364) that was used to assess expression of selected snoRNA and RP genes in endometriosis lesions (n=21, represented as E) and normal endometrium (n=16, represented as N). **P<0.05; **P<0.01; ***P<0.001.*

To study the biological relevance of *MIR196A2* polymorphisms in endometrial cells, cells transfected with either miR196a2-T or miR196a2-C vectors were subjected to gene expression profiling by microarray analysis. The miR196a2-C vector induced > 1.5 fold changes in a majority of known C/D snoRNAs (FIG. 3A, *upper).* Nearly half the known human RPs were also moderately increased (fold change > 1.3; FIG. 3A, *lower).* To confirm the microarray data, we assessed snoRNAs and RPs with fold changes > 2 by qPCR. Most snoRNAs showed expression patterns consistent with the microarray data, although SNORD54 and SNORD45A levels were lower as measured by qPCR (FIGS. 3B-3C). Among the highly expressed RPs, ribosomal protein large P2 (RPLP2), RPL27A, RPS27 (also known as metallopanstimulin-I, MPS-I) and 60S ribosomal protein L38 (RPL38), were validated to be regulated by the miR196a2-C vector.

To define the clinical significance of our findings, microarray data from the GEO databank (accession number: GSE6364) were utilized to analyze expression of the selected snoRNAs and RPs in clinical endometriosis lesions and normal endometrium. Six out of eight RPs were found to be upregulated in endometriosis tissues (FIG. 3D). Due to limitations of the probe-set design, small nucleolar RNA *C*/*D* box 116 (SNORD 116) was the only selected snoRNA gene found in the dataset with higher levels in endometriosis tissues (represented as E) compared to controls (represented as N). Among the selected genes, SNORD 116, RPLP2, RPL38 and 40S ribosomal protein S28 (RPS28) were most elevated ones in endometriosis patients (FIG. 3D, represented as E), which was consistent with our *in vitro* experimental findings using miR196a2-C vector. These results indicate an overall activation of ribosome biogenesis during endometriosis development.

### 3. Ribosome biogenesis upregulation triggers endometriosis progression

Ribosome biogenesis is the greatest energetic and metabolic expenditure that takes place in the cell nucleolus, especially in cancer cells. Structural-functional studies have revealed that nucleolar abnormalities correlate with cancer development and represent an adaptation to the new metabolic characteristics acquired by transformed cells. Therefore, the activation of ribosome biogenesis might be a driving force triggering malignant transformation during endometriosis progression.

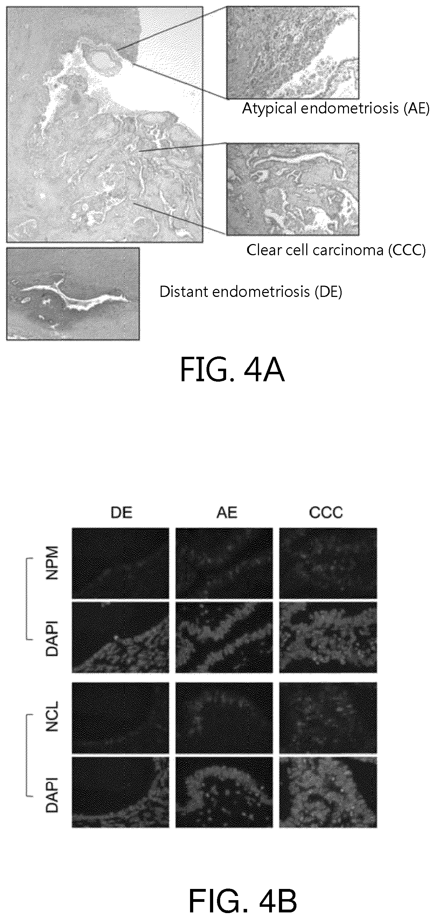

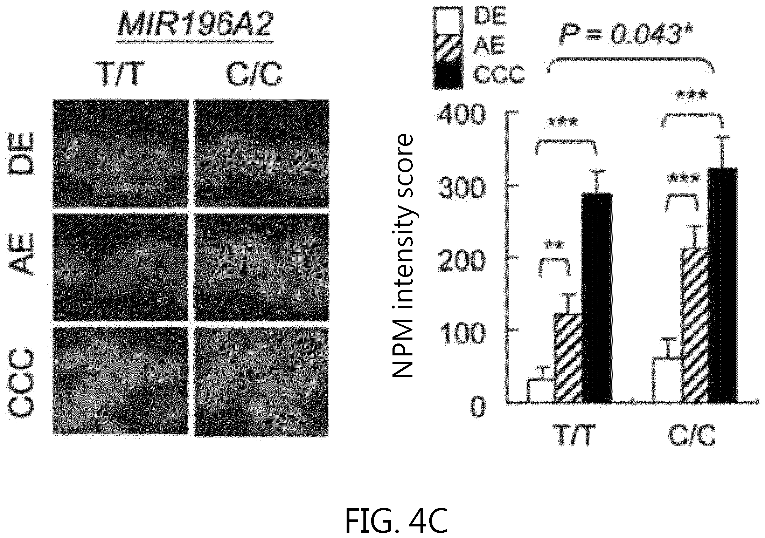

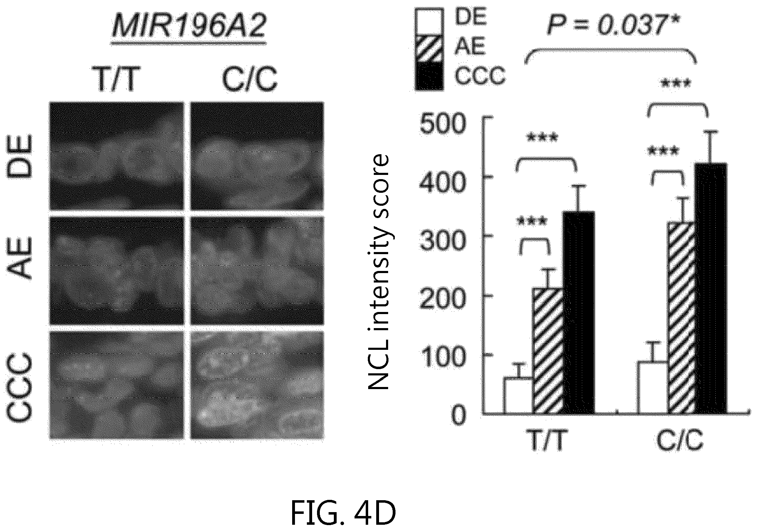

Please refer to FIGS. 4A to 4D, which illustrate ribosome biogenesis upregulation during endometriosis progression. FIG. 4A is depicted to tissue sections with contiguous atypical endometriosis and ovarian clear cell carcinoma that were genotyped (5 blocks for *C*/*C* and 3 blocks for *T*/*T* at rs11614913 in *MIR196A2).* FIG. 4B is depicted to tissue sections prepared for anti-NPM and anti-NCL staining. The representative staining images that were from tissue blocks with *C*/*C* genotype at rs11614913 in *MIR196A2.* Staining was scored as aforementioned. The scores of all staining images were represented as means of 100 nucleoli with standard variations. FIGS. 4C and 4D is depicted to magnified images indicate nucleolus (anti-NPM)(FIG. 4C) and DFC (anti-NCL)(FIG. 4D) staining. Distant endometriosis from the same patient was used as the control of FIGS. 4A to 4D. **P<0.05; **P<0.01; ***P<0.001*.

To test this hypothesis, five clear cell ovarian carcinomas carrying the risk *C*/*C* genotype at rs11614913 in *MIR196A2* and three samples carrying the reference *T*/*T* genotype were collected in this Example for immunofluorescence staining (FIG. 4A). In this Example, total active nucleoli were detected using anti-nucleophosmin (NPM) antibodies, and the dense fibrillary component (DFC), a region with highly active ribosome biogenesis, was detected using anti-nucleolin (NCL) antibodies. The results indicated that contiguous atypical endometriosis adjacent to cancer tissues had greater NCL and NPM staining intensities compared to distant endometriosis lesions (FIGS. 4B-4D). Consistent with this, cancerous tissue nucleoli had greatly enlarged total areas (anti-NPM) with activated ribosome biogenesis (anti-NCL) (FIGS. 4B-4D). Of note, the tissue blocks carrying the *T*/*T* genotype showed a weaker staining intensities than those carrying the *C*/*C* genotype. However, the increasing patterns in are similar between these two groups (FIGS. 4C-4D). The data provide evidence that increased nucleoli and enlarged DFC morphology indicate an unfavorable transformation from endometriosis to atypical endometriosis and finally ovarian cancer.

Functional MiRSNPs impact human disease, including cancer development. The present application assessed six cancer-related MiRSNPs and found that genetic variations at rs11614913 in *MIR 196A2* are associated with endometriosis development and progression. The rs11614913 C allele correlated with a greater tendency for patients to develop infertility and severe pain. Functional characterization in endometrial cells demonstrated a role for this risk allele in ribosome biogenesis via regulating expression of multiple snoRNAs and RPs. These snoRNAs and RPs were generally upregulated in endometriosis lesions as compared to normal endometrium, suggesting that active ribosome biogenesis in cell nucleoli drives endometriosis. Immunofluorescent staining against NPM and NCL further confirmed that changes in nucleolar integrity correlate with aggressive progression from endometriosis to atypical endometriosis and clear cell ovarian cancer. Treatment with CX5461, an RNA polymerase 1 inhibitor, inhibited cell proliferation and migration in ovarian clear cells that possess the risk C/C genotype of rs11614913, and triggered cell cycle arrest at G2/M phase and apoptosis. To our knowledge, this is the first report to address the roles of *MIR196A2* genetic variants in endometriosis development and malignant transformation.

The functional SNP rs11614913 in *MIR196A2* is associated with cancer development, including lung and breast cancers. Although some discrepancies exist across different cancer types and ethnic groups, most studies associate the CC or CT genotypes at rs11614913 with poorer patient outcomes, indicating the C allele as the risk allele. Consistent with the results of Examples, rs116l49l3-C is reportedly more structurally stable than rs116149l3-T, and is correlated with increased mature miR196a2 in clinical specimens. *MIR196A2* is located in the *HOXC* cluster region on chromosome 12. Nearly one-third of known or putative miR196a2 targets (Table 5) are members of the Hox gene family, which encodes homeodomain-containing transcription factors essential for embryonic development. Hox proteins participate in cell division, adhesion/migration and apoptosis, and dysregulation of these proteins has been linked to endometriosis development, embryo implantation and malignancy. However, most Hox proteins are sensitive to steroid hormones, including the clinically used hormone drugs, and their levels change along with the menstrual cycle. This might explain why we did not see consistent Hox expression patterns in clinical specimens.

On the other hand, an overall increase in snoRNAs and RPs as the downstream effectors of miR196a2 suggests enhanced ribosome activity crucial for cell proliferation and the expansion of endometriosis tissue. These data indicated that rs116149l3-C enhanced expression of SNORD 116, RPLP2, RPS27, RPS25, RPL26, RPL38 and RPS28, all of which were upregulated in clinical specimens. Florescent staining against both NPM (active nucleoli) and NCL (DFC regions in the nucleoli) revealed that ribosome biogenesis was more active in contiguous atypical endometriosis than in distant endometriosis, and greater staining patterns can be found in cancerous tissues. Thus, the present application suggests the point that active ribosome biogenesis could be a driving force for malignant transformation during endometriosis development and progression.

Previous studies support this application that overexpression of RPs contributes to cell transformation and could be utilized as prognostic markers for human cancers. Ribosomal P protein (RPLP0, RPLP1, RPLP2) expression was previously shown to correlate with invasiveness and metastasis in gynecologic tumors. Although limited information is available regarding the functions of SNORD116, a C/D box snoRNA that controls the 2'-O-ribose methylation of rRNAs, accumulating evidence implicates snoRNAs in the control of cell fate and carcinogenesis through a bypassing of ribosomal/oncogenic stress responses. For example, upregulation of C/D box snoRNAs was reported as a common feature in breast and prostate cancers.

In addition to their key functions in ribosome assembly and protein synthesis, snoRNAs and RPs play novel roles outside cell nucleoli, regulating the activity and function of other oncogenes or tumor suppressors. Several downstream effectors of rs l16l49l3-C, including RPS27, RPL26, RPS25 and RPL26, participate in the MDM2-p53 feedback loop upon ribosomal/oncogenic stress. Disruption of rRNA synthesis and editing/processing, such as by chemical inhibition of RNA polymerase I, triggers MDM2 degradation and stabilizes/activates p53, leading to cell apoptosis or senescence. Similarly, specific siRNAs against C/D box snoRNAs suppressed cell cycle progress and reduced tumor growth by activating p53. With emerging roles for RNA processing in cancer development, targeting rDNA transcription and the nucleolus is a feasible cancer treatment strategy, and has shown efficacy against hematological malignancies.

Notably, human cancers exhibit differential sensitivity to anti-RNA polymerase 1 therapy, depending largely on *TP53* status. Genetic analysis has shown that *TP53* mutations rarely occur (∼10%) in endometriosis-associated ovarian cancers, and are considered as late genetic events during endometriosis progression if they occur. The present application indicates enhanced ribosome biogenesis activity during endometriosis development, and this activity is more pronounced during the malignant transition. This suggests that anti-RNA polymerase I therapy may be efficacious for treating endometriosis and associated ovarian cancers. Additionally, genetic variations at rs11614913 in *MIR196A2,* along with upregulation of snoRNAs and RPs associated with ribosomal biogenesis, may be useful prognostic indicators in endometriosis patients.

### EXAMPLE 3

### 1. Source of Specimens

Twenty-three endometriosis patients were diagnosed pathologically in China Medical University Hospital (CMUH), whose conditions were also compared to clinical reports of open surgical or laparoscopic surgery. The endometriosis was classified into four stages of severity as defined by the criteria of the American Society of Reproductive Medicine (ASRM). Healthy specimens were obtained from ten healthy women, which were examined physiologically and periodically by Kaohsiung Veterans General Hospital. Informed consent was obtained from all participants of the study. All specimens were placed onto smear slides, dehydrated by ethanol and stored in a lyophilized form before the following experimentations.

### 2. Immunofluorescence Staining

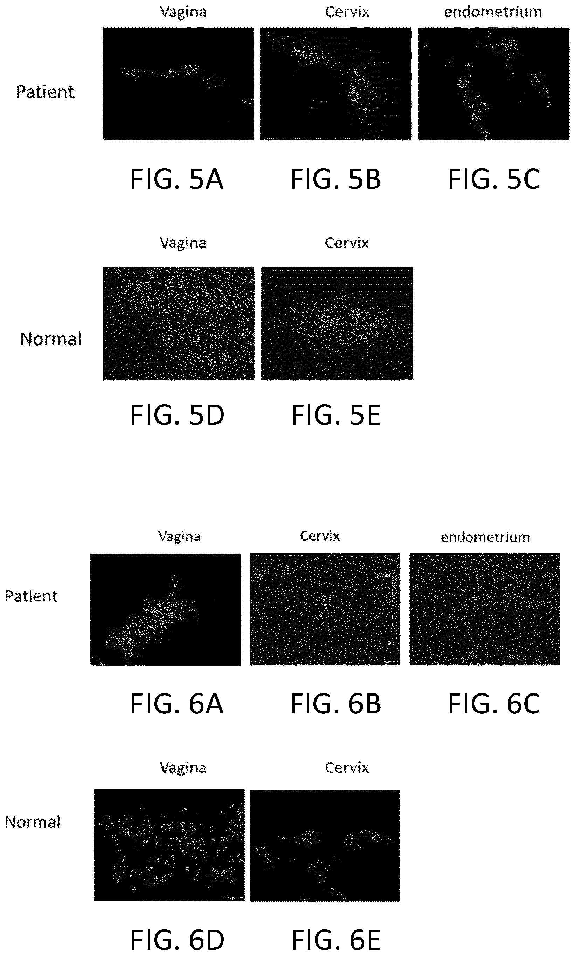

The smear slides were rehydrated by sequential immersion in 100% ethanol for 5 minutes, 95% ethanol for 3 minutes, 75% ethanol for 3 minutes, 50% ethanol for 3 minutes and water for 5 minutes. Next, the smear slides were incubated in blocking buffer (containing 0.1% Triton X-100 and 1% FBS; Sigma) under room temperature (approximately in 5 to 40 degrees Celsius) for permeabilizing cells and blocking non-specific antigens. After rinsing thrice of Tris-buffered saline with Tween 20 (TBST), the smear slides were reacted with primary antibody under room temperature for 4 hours. And then, after rinsing thrice of TBST, the smear slides were reacted with secondary antibody under room temperature for 1 hours and the nuclei were stained with 4',6-diamidino-2-phenylindole (DAPI) for 1 minute. Afterwards, the smear slides were sealed and examined by fluorescent microscopy, as shown in FIGS. 5A to 10E.

### 3. Difference of Ribosomal Protein Expression in Reproductive Tracts Between Endometriosis Patients and Healthy Women

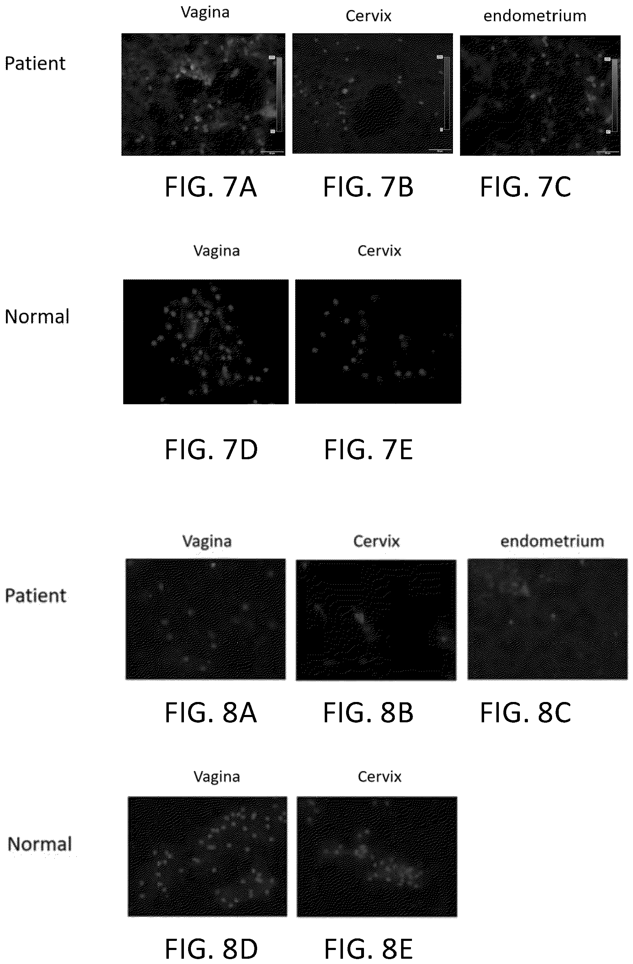

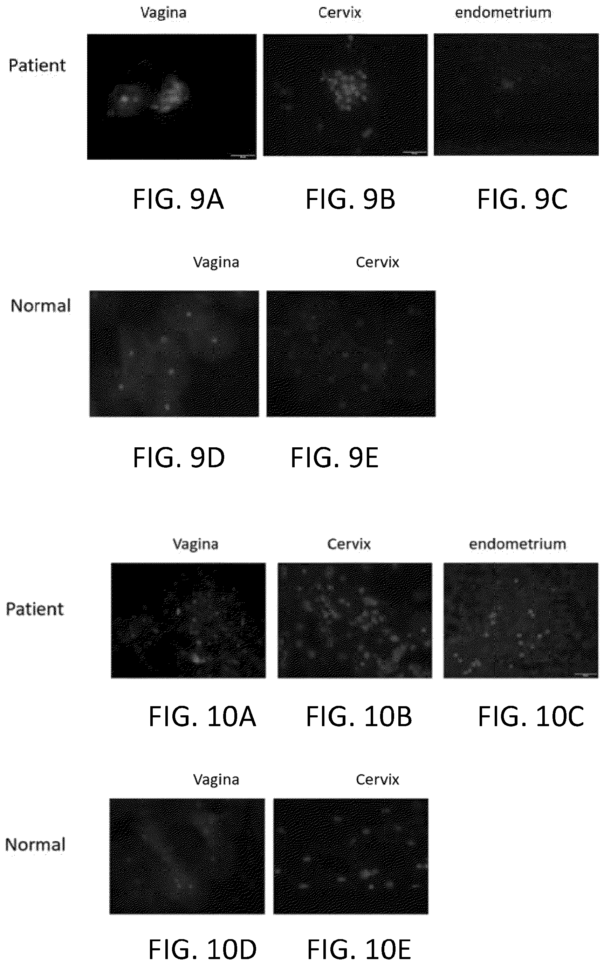

Smears of the cervix and vagina were examined by immunofluorescence staining for confirming the expressions of ribosomal proteins, and all results were shown in FIGS. 5A to 10E. Reference was made to FIGS. 5A to 10E, which showed the expressions of nucleolin (FIGS. 5A to 5E), RPL38 (FIGS. 6A to 6E), RPS13 (FIGS. 7A to 7E), RPLP2 (FIGS. 8A to 8E), RPS27 (FIGS. 9A to 9E) and RPS25 (FIGS. 10A to 10E) in the cervix and vagina of patients and healthy women according to an embodiment of the present invention. In the statically significant results of FIGS. 5A to 10E, the expressions of nucleolin, RPL38, RPS13, RPLP2, RPS27and RPS25 in the cervix and vagina of patients were significantly higher than those of healthy women, and the difference between the two group was statically significant. These results indicated that endometriosis progression was associated with the ribosome biogenesis upregulation.

### 4. Relationship Between Ribosomal Biosynthesis and Severity of Endometriosis

The members in the patient group were the endometriosis patients who needed surgery. Therefore, in this EXAMPLE, the influence of upregulation of ribosomal biosynthesis on endometriosis was further assessed.

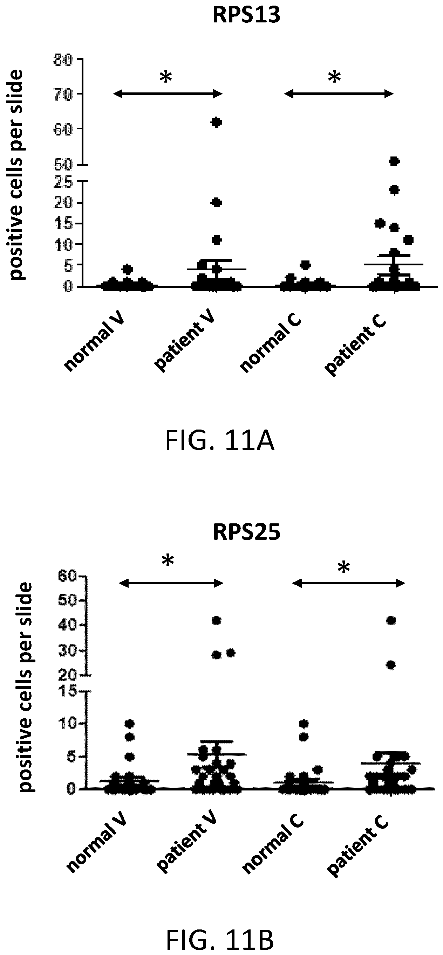

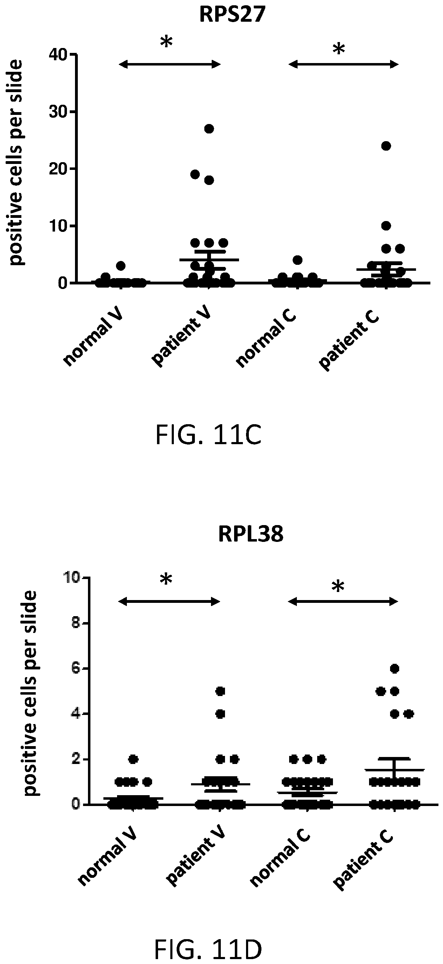

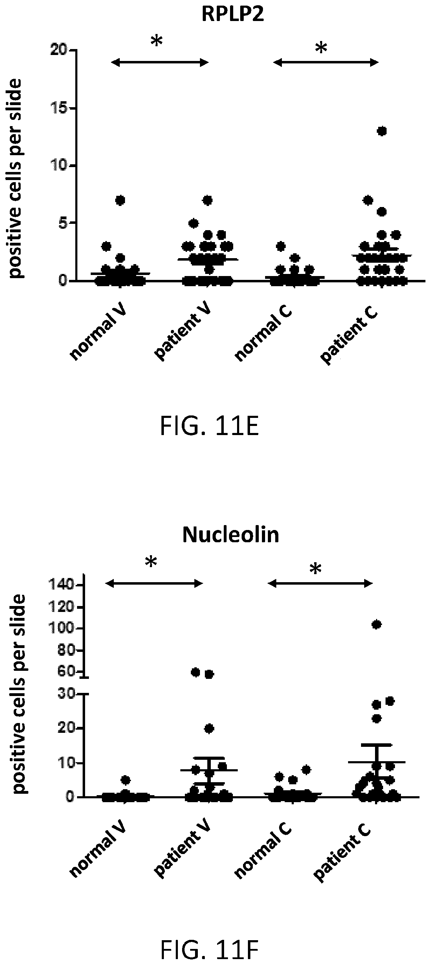

All patients were examined by clinical criteria of endometriosis. Those examination results were classified by disease stages, pain scores and CDS obliteration, statistically analyzed by commercial software, and shown in FIGS. 11A to 13.

The biomarkers-positive cells per slide of the endometriosis patients and healthy (normal) women were shown in FIGS. 11A to 11F.

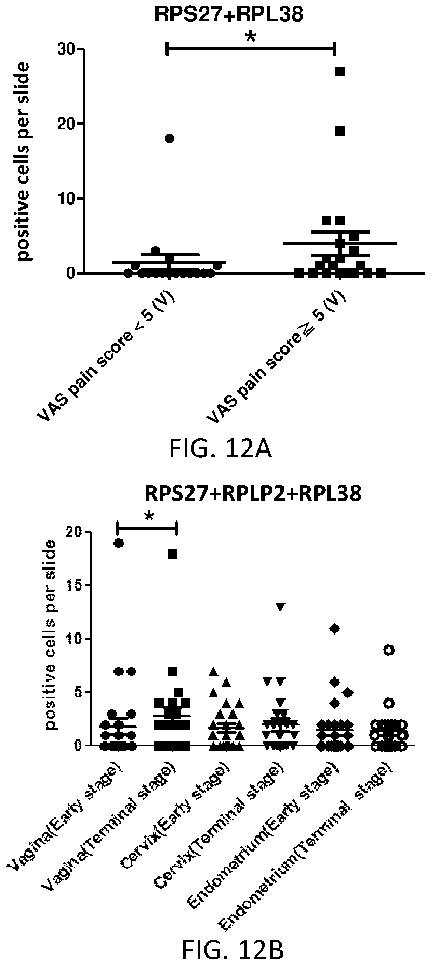

The pain score of the patients were determined by visual analog scale (VAS) and its corresponding visual analog pain scale, which were psychometric scales that were generally used in hospitals and clinics by doctors to conduct pain scale surveys to understand varying degrees of pain or discomfort experienced by a patient. Scores are based on self-reported measures of symptoms that are recorded with a single handwritten mark placed at one point along the length of a 10-cm line that represents a continuum between the two ends of the scale-"no pain" on the left end (0 cm) of the scale and the "worst pain" on the right end of the scale (10 cm). In this EXAMPLE, all patients were divided into two groups as determined by the VAS pain scores less than 5 (cm) (i.e. VAS pain score < 5) or the VAS pain scores equal to or greater than 5 (cm) (i.e. VAS pain score ≥ 5). The result of the pain scores of the endometriosis patients were shown in FIG. 12A.

The disease states of the endometriosis patients were determined according to the revised American Fertility Society classification (r-AFS classification), which was the most widely used staging system of endometriosis, for providing limited predictive ability for pregnancy after surgery. The early and terminal states of endometriosis in the vagina, cervix and endometrium were determined by r-AFS classification. The disease states of the endometriosis patients corresponding to RPS27-, RPLP2- and RPL38- positive cells per slide were shown in FIG. 12B.

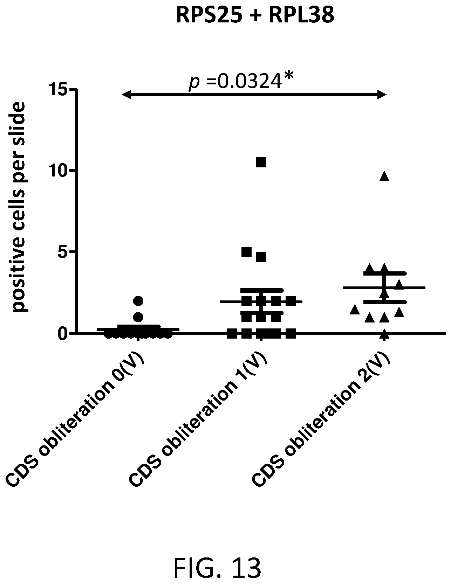

The cul-de-sac (CDS) obliteration in the endometriosis of the patients were determined by Enzian classification, which enables clinicians to classify deeply infiltrating endometriosis (DIE). This classification addressed the issues of the posterior compartment DIE and bladder (anterior) and ureteral (lateral) compartments. The level 0 of CDS obliteration showed no endometriomas. The level 1 of CDS obliteration showed endometriomas less than 1 cm in diameter. The level 2 of CDS obliteration showed endometriomas with 1-3 cm in diameter. The results of the CDS obliteration of the endometriosis patients were shown in FIG. 13.

Reference was made to FIGS. 11A to 13, which illustrated the cells expressing biomarker-positive cells per slide (FIGS. 11A to 11F), VAS pain scores related with the biomarkers (RPS27 and RPL38) (FIG. 12A), different disease stages related with the cells expressing biomarker (RPS27, RPLP2 and RPL38)- positive cells (FIG. 12B) and the CDS obliteration with the biomarkers (RPS25 and RPL38) (FIG. 13) between the endometriosis patients and healthy (normal) women. In FIGS. 11A to 13, the results of vagina (V) and cervical (C) samples were whether they had statistical significances or not. Asterisks were assigned to represent different levels of statistical significances, p values less than 0.05 were given one asterisk ("*"), p values less than 0.01 were given two asterisks ("**"), and p values less than 0.001 were given three asterisks ("***").

The results of FIGS. 11A to 13 showed a trend that the cells sampled from vagina had more expression of ribosomal proteins depending on the increased severity of clinical criteria of endometriosis patients. In the past reports, rectovaginal endometriosis (RVE), one of the most severe form of deep infiltrating endometriosis (DIE), could infiltrate the vagina, rectum and rectovaginal septum. As aforementioned, ribosomal biosynthesis upregulation promoted worse endometriosis, resulting in more severe infiltration and adhesion.

Currently, most tests for clinically screening endometriosis are invasive and difficultly popularized because of adverse influence on the patients' willingness to undergo these tests. In some previous studies, upregulation of ribosome biosynthesis resulted from genetic mutations can worsen endometriosis. Therefore, the present invention evidences the possibility of ribosomal proteins as targets for screening endometriosis. In the invention, five ribosomal proteins that affect the mortality of endometrial cancer are screened from the Cancer Genome Atlas (TCGA) as the targets, the expression of ribosomal proteins in smears of endometriosis patients (vaginal, cervical and endometrium) and healthy women (vagina, cervix) are detected by fluorescence Immunostaining. The results are showed that the five ribosomal proteins (RPS13, RPS25, RPS27, RPLP2, RPL38) and nucleolin are associated with endometrial cancer and have significant difference. Moreover, the relationship between the deterioration of endometriosis and the upregulation of ribosomal protein is supported by the aforementioned results, which show that the amount of ribosomal protein expression in vaginal cells increases with the increased severity of clinical diagnostic indicators. In addition, these specimens are obtained from the endometriosis patients who have severe infiltration, adhesion and undergone surgery. The upregulation of ribosomal protein may possibly affect the infiltration and adhesion of endometriosis. The expression of ribosomal proteins may be detected as a tool for screening endometriosis in future.

It is worth mentioning that, it is still unknown how genetic variation in a miRNA can promote upregulation of genes involved in ribosome biogenesis, especially C allele at rsl16l49l3 can form more stable and abundant mature miR196a2. In addition, the selected snoRNAs and RPs in this study are not putative direct targets of miR196a2 based on the degree of complementarity between the target site and the miRNA. Interestingly, recent studies did provide evidence that miRNAs can promote specific gene upregulation through direct or indirect mechanisms. These clues support the possible involvement of other factors in miR196a2-mediated upregulation of ribosome biogenesis which is worth a further investigation.

In summary, it is necessarily supplemented that, specific SNP sites, expression of specific genes, specific criteria for classification of clinical disease severity, specific analysis models or specific evaluating methods are exemplified for clarifying the biomarker, the probe, the primer pairs, the kit and the method of non-invasively and qualitatively determining severity of endometriosis. However, as is understood by a person skilled in the art, other specific SNP sites, expression of other genes, other criteria for classification of clinical disease severity, other analysis models or other evaluating methods can be also adopted in the biomarker, the probe, the primer pairs, the kit and the method of non-invasively and qualitatively determining severity of endometriosis of the present invention.

According to the embodiments of the present invention, the biomarker and the method of non-invasively and qualitatively determining severity of endometriosis of the present invention are advantageous to establish an association between the biomarker and severity of endometriosis, so as to accurately determine the severity of endometriosis through detecting the biomarker, thereby specifically being applied to a probe, primer pairs, antibodies and a kit for in vitro testing a panel of genes in Pap smear samples for endometriosis.

Although the present invention has been described in considerable detail with reference to certain embodiments thereof, other embodiments are possible.

## Claims

1. A kit for in vitro testing a panel of genes in Pap smear samples for endometriosis, comprising:
at least nine antibodies, wherein each of the nine antibodies respectively recognizes one protein expressed by the panel of the genes or SNP sites, the panel includes at least nine different genes, and the panel is selected from the group consisting of SNORD116 gene, RPLP2 gene, RPL26 gene, RPL38 gene, RPS25 gene, RPS27 gene, RPS28 gene, rs11614913 and/ or rs1834306.

2. A method of non-invasively and qualitatively determining severity of endometriosis, comprising:
providing a kit for in vitro testing a panel of genes in the biological sample for endometriosis, wherein the kit comprises at least nine antibodies, wherein each of the nine antibodies respectively recognizes one protein expressed by the panel of genes or SNP sites, the panel includes at least nine different genes, and the panel is selected from the group consisting of SNORD116 gene, RPLP2 gene, RPL26 gene, RPL38 gene, RPS25 gene, RPS27 gene, RPS28 gene, rs11614913 and/ or rs1834306;
establishing a correlation model, comprising:
detecting a plurality of reference biological samples by the kit, so as to obtain a plurality of first risk data, wherein the first risk data comprise a plurality of SNP sites and first expressions of a plurality of genes or proteins epigenetically associated with the SNP sites, SNP accession numbers of the SNP sites comprises rs11614913 and rs1834306, and the genes or the proteins are selected from the group consisting of SNORD116, RPLP2, RPL26, RPL38, RPS25, RPS27, and RPS28; and
establishing a correlation between the first risk data and a plurality of severe statuses of endometriosis, so as to correspond one of the SNP sites with one of the genes or the proteins, one of the first expressions and/or one of the severe statuses;
determining an expression of the biomarker in a test biological sample according to the correlation model, comprising:
non-invasively detecting the test biological sample, so as to obtaining a plurality of second risk data, wherein the second risk data comprises the SNP sites and second expressions of the genes or the proteins epigenetically associated with the SNP sites;
comparing the second risk data to the first risk data, for determining that the second risk data matches one of the first risk data or not, and obtaining a corresponding significance difference (P value); and
determining that the test biological sample has the one of the severe statuses when the second risk data matches the one of the SNP sites and the one of the genes or the proteins, and the corresponding significance difference (P value) is smaller than 0.05.

3. The method of claim 2, wherein the reference biological samples and the test biological sample are Pap smear samples comprising an *ex vivo* sample of blood or tissue.

4. The method of claim 2, wherein a genotype of the rs11614913 comprises C allele, CC genotype or CT genotype.

5. The method of claim 2, wherein a genotype of the rs1834306 comprises A allele or AA genotype.

6. The method of claim 2, wherein the first expressions are upregulated.

7. The method of claim 2, wherein the severe statuses comprises a clinical stage, a CA125 level and a pain score of the reference biological sample.


SEQUENCE LISTING

<110> National Sun Yat-Sen University, Taiwan

<120> KIT FOR IN VITRO TESTING PANEL OF GENES IN PAP SMEAR SAMPLES FOR
      ENDOMETRIOSIS AND METHOD OF NON-INVASIVELY AND QUALITATIVELY
      DETERMINING SEVERITY OF ENDOMETRIOSIS

<130> 203315EP

<150> US 16/726,546
<151> 2019-12-24

<160> 6

<210> 1
<211> 51
<212> DNA
<213> Artificial sequence

<220>
<223> probe for detecting SNP site rs11614913 (C_31185852_10)

<400> 1
ttttgaactc ggcaacaaga aactgyctga gttacatcag tcggttttcg t      51


<210> 2
<211> 51
<212> DNA
<213> Artificial sequence

<220>
<223> probe for detecting SNP site rs1834306 (C_11483095_10_F)

<400> 2
agcaatatct tctatgttct ccccarcgtg cttcccttgg tttccacttt t      51


<210> 3
<211> 51
<212> DNA
<213> Artificial sequence

<220>
<223> probe for detecting SNP site rs2910164 (C_15946974_10)

<400> 3
catgggttgt gtcagtgtca gacctstgaa attcagttct tcagctggga t      51


<210> 4
<211> 51
<212> DNA
<213> Artificial sequence

<220>
<223> probe for detecting SNP site rs7372209 (C_29123986_10)

<400> 4
agaattagga gagaaattaa tccttygtac cacgtgactg taagcatgac t      51

<210> 5
<211> 51
<212> DNA
<213> Artificial sequence

<220>
<223> probe for detecting SNP site rs895819 (C_11483095_10_F)

<400> 5
agcaatatct tctatgttct ccccarcgtg cttcccttgg tttccacttt t       51


<210> 6
<211> 51
<212> DNA
<213> Artificial sequence

<220>
<223> probe for detecting SNP site rs6505162 (C_11613678_10)

<400> 6
tgaggcccct cagtcttgct tcctamcccg cgcttgagtt tctccccgct t       51